# EUROPEAN PATENT SPECIFICATION

(11) **EP 4 171 739 B1**
(45) Date of publication and mention of the grant of the patent: **27.11.2024**
(21) Application number: 21745585.6
(22) Date of filing: 28.06.2021
(51) Int. Cl.: A61K 9/00, A61K 31/732, A61K 47/36, A61P 1/00, A61P 11/00, A61K 45/06

(54) **LOZENGE**
LUTSCHTABLETTE
PASTILLE

(30) Priority: 30.06.2020 US 202063045947 P
(43) Date of publication of application: 03.05.2023
(73) Proprietor: Haleon US Holdings LLC, Wilmington, DE 19808 (US)
(72) Inventor: ENGLAND, Kenneth Earl, Richmond, VA 23220 (US); PATEL, Shivangi Akash, Richmond, VA 23220 (US); YANG, Chue Hue, Mooresville, NC 28115 (US)
(74) Representative: Haleon Patent Department
(86) International application number: PCT/US2021/039310
(87) International publication number: WO 2022/005932

(56) References cited:
- DE-A1- 102010 011 964
- US-A- 3 485 920
- US-B1- 6 258 383

## Description

### Technical Field

This invention relates to a sweet lozenge for the delivery of pectin to treat sore throats. The lozenges of the present invention utilise pectin as the sole active to treat the discomfort. The invention provides an optimised formulation to achieve superior soothing performance over current products.

### Background

Sore throat remedies are extremely popular among consumers. They are a treatment that requires no input from health workers and therefore can be used quickly as required when the need arises.

There are many different products that can be used to treat sore throats for consumers. These include different dosage forms and a variety of different actives and mechanisms of action to achieve the treatment of soreness.

This invention is generally related to throat lozenges and more specifically to throat lozenges that utilise pectin as the active ingredient.

Pectin works as a demulcent. A demulcent is an agent that forms a soothing film over a mucous membrane, relieving minor pain and inflammation of the membrane.

However, demulcents generally last for around 30 minutes or so, as the normal actions of the throat (swallowing or breathing or any eating or drinking) gradually remove the protective layer. This means that effective relief requires a continual replenishment of the film. This favors the delivery of these active agents ("actives") in sweet lozenges that can be consumed steadily throughout the day.

Demulcents are sometimes referred to as mucoprotective agents. As well as pectin, natural demulcents include, glycerin, honey and syrup. Methylcellulose, propylene glycol, and glycerine are good examples of synthetic demulcents.

Such demulcents are commonly used in over the counter (OTC) remedies to treat sore throats.

Pectin is a heteropolysaccharide commercially derived from the cell wall of higher plants.

It is composed of partially methylated polygalacturonic acid units linked in the positions 1-4.

The carboxylic group of the constituents of pectin can exist in the form of esters, free acids, ammonium, potassium or sodium salts or as acid amides.

Under US Food and Drug Administration ("FDA") regulations, pectin qualifies as a GRAS (generally recognized as safe) food substance.

Lozenges are popular delivery vehicles for oral actives. They are usually sweet to encourage and sustain consumption. Lozenges range from soft jelly like gummies to hard solid, sugar based, candies.

Pectins are found in many commercial lozenges. Examples include Luden's^{®} range of lozenges, Halls^{®} Breezers and Smith Bros'^{®} Throat drops.

Delivery of pectin in the amounts and timings needed to effectively sooth a sore throat can be problematic using a lozenge treatment product.

For effective soothing and treatment of a sore throat, a layer of pectin needs to be built up over the sore tissue. This requires a balance in terms of quantity of the pectin available, ease of release from the lozenge, and the delivery time frame.

Soft gummies can readily be loaded with pectins but are usually designed to encourage chewing and rapid consumption. This is a good format for the delivery of actives such as vitamins and medicaments/actives that require rapid systemic reach.

However, because a gummy is usually consumed in much less than a minute, it is generally a poor format for the delivery of a topical active for the throat/oral cavity, as any pectin (or other active) contained is not fully released from the rest of the gummy prior to swallowing.

Most of the pectin or other actives in a soft gummy pass straight through to the stomach without being able to coat the mucal tissue as required.

Hard candy lozenges are more the ideal form for the slow release of actives for use in the mouth and throat. The hard candy body can take many minutes to dissolve in the mouth. And this allows for the steady release of actives. The hard body discourages early swallowing too.

However, the hardness also sometimes encourages crunching behaviour in some consumers. This is problematic as it also means, like with the soft gummies, that much of the pectin contained would be lost to the stomach.

Unfortunately, it is also difficult to achieve the levels of pectin required to treat a sore throat in a hard sweet. The solubility/mixability of pectin in the hard sugar candy form is low.

This can be seen in the fact that hard candy commercial throat lozenges containing pectin are usually very low loaded. Between 2 and 7 mg of pectin per lozenge is common for commercial hard candy throat lozenges.

This level of pectin is really too low for effective demulcent capability. Since demulcent performance requires a physical coating of the tissue to provide relief. More demulcent material is required to give a satisfactory coating to the inflamed throat tissues. Patent document US 3,485,920 discloses analgesic lozenges comprising pectin.

It is an object of the present invention to provide a much more effective throat lozenge for the treatment of sore throats.

### Summary

In its broadest form, the first aspect of the present invention encompasses a lozenge comprising:
a) 1.4-1.8 % by weight pectin;
b) 78-88 % by weight sweeteners;
c) 0.1-1.5 % of an organic acid; and
d) 10-19 % by weight of water.

Further it is preferred that the lozenge does not comprise gelatin, starches or any other gelling or rheology modifying agents.

In a further aspect the lozenge has a total weight of between 2 and 6 grams.

In a further aspect the lozenge comprises at least 40 mg of pectin.

In a further aspect the sweetener comprises between 82-86 % by weight of the lozenge.

In a further aspect the sweetener is an artificial sweetener.

In a further aspect the sweeteners comprise one or more of fructose, sucrose, lactose, glucose and mixtures thereof.

In a further aspect the sweetener is a combination of sucrose and corn syrup and the sucrose and the syrup may be present in a weight ratio of 20:80 to 80:20.

In a further aspect the organic acid component comprises between 0.5-1% by weight of the lozenge.

In a further aspect the organic acid comprises citric acid, salts of citric acid and mixtures thereof.

In a further aspect the water comprises between 12-17 % by weight.

In a further aspect the lozenge has one or more of a flavouring component, a dye, preservative or mixtures thereof.

In a further aspect the lozenge has a hardness of between 8000 and 20,000 grams as measured by the TPA test.

In a further aspect the lozenge further comprises at least one further active selected from a group consisting essentially of menthol, guaifenesin, acetaminophen, benzocaine, phenylephrine HCl, dextromethorphan HBr, and mixtures thereof.

In a further aspect of the invention the lozenge consists essentially of:
1.6 % by weight pectin;
81-83 % by weight sucrose and glucose;
14-17 % by weight water;
0.9-1.10% by weight citric acid and/or citric acid salts and/or mixtures thereof; and
0.1-0.5 % by weight flavorings.

And in a further aspect the weight ratio of sucrose to glucose of this lozenge is between 60:40 and 40:60. And further the lozenge has a total weight of between 3 and 4 grams.

The invention additionally encompasses a composition for use in a method of treating sore throats comprising the application of a lozenge described in the first aspect to the invention to the oral cavity of a patient in need and further allowing the lozenge to dissolve over a period of at least 5 minutes

In a further aspect the composition for use in a method of treatment discloses that the lozenge dissolves over a period of at least 10 minutes.

### Definitions

All percentages are weight percentages based on a total weight of the composition unless otherwise stated.

The term "comprising" for the purposes of the present specification means including the following features, but not limited to them. An open-ended construction.

The term "consisting of" for the purposes of the present specification means including the following terms only. A closed construction.

The term "consisting essentially of" for the purposes of the present invention is equivalent to "consisting of" with the exceptional possibility of the additional inclusion of small amounts of non-essential ingredients. For the purposes of the present invention "consisting essentially of" means less than 5 % by weight of the composition may be non-defined, non-essential ingredients. These may include such minor ingredients as dyes, flavours, preservatives etc.

For the purposes of the present specification, any disclosure of a composition or formulation *comprising* the following features may be assumed to disclose the self-same composition or formulation *consisting of,* or *consisting essentially of,* the same features.

### Detailed Description

Applicants have surprisingly found that by carefully controlling the levels of pectin, sweetener, acid and water an optimal balance can be struck that provides a lozenge with high levels of pectin and just the right levels of hardness to provide a lozenge with an ideal release profile in the mouth.

This ideal hardness level naturally encourages the consumption of the lozenge by sucking rather than chewing. It is not too soft and not too hard, to discourage crunching.

The composition of the lozenges also contains enough moisture to keep pectin levels high enough to make for very effective throat soothing.

The lozenges of the present invention have been optimised for use with pectin as the sole active and demulcent.

Pectin for the purposes of the present invention may be used in a variety of purities and grades. Pectin is a natural product and will have inevitably contain minor levels of natural contaminants. It is preferable, for the purposes of the present invention to use the highest purity grades of pectin.

Gelatin, for example, is often used in lozenge candies sweets to control texture, provide elasticity in gummies. Starches and other gelling agents are also often utilised to form perform similar functions.

The lozenges of the present invention do not include gelatin or starches or any other gelling or rheology modifying agents. By a careful control of the ratio of the limited ingredients used, the present invention provides candy lozenges that are completely free of other modifying agents.

As such the lozenges of the present invention are highly suitable for vegetarians.

Minor ingredients such as colourants, preservatives and flavours may still be included however in some cases.

In its broadest extent the lozenge of the present invention comprises or consists essentially of:

**Table 1**

| **Ingredient** | **% Weight** |
|---|---|
| Pectin | 1.4-1.8 % |
| Sweetener | 78-88 % |
| Acid | 0.1-1.5 % |
| Water | 10-19 % |

In principal there is no reason to limit the upper level of pectin to this range in a finished lozenge. However as pectin acts as a gelling and binding agent, to achieve the right level of hardness in the lozenge while maintaining efficient conditions in manufacture, there is a practical upper limit of less than 2% pectin in the optimised formulations of the present invention to achieve a lozenge with the desired properties.

Using a level of pectin greater than 2% by weight means that the formulation is both difficult to handle and produces an uneven final product. Higher levels of pectin also raise the gelling temperature and requires too much energy (heat) to stay liquid. It is also extremely difficult to mix evenly and risks that the formulation solidifies too early and blocks process lines)
The range of pectin for the present invention is between 1.4 and 1.8 %, more preferably between 1.5 and 1.7 % by weight and most preferably about 1.6% by weight of the lozenges.

The amount of pectin required in a lozenge for effective relief of soreness will depend on a variety of factors. The area of the sore or inflamed tissue, the rate of release of pectin from the lozenge, the rate the mucal protective layer is reduced (coughing or swallowing), the rate of consumption of the lozenges in the mouth (dissolution time) and the quantity of pectin in the lozenge.

These factors will clearly vary from person to person. But the applicants have found that for effective treatment of inflamed throat tissue at least 30-40 mgs of pectin per lozenge is required for most adults. Preferably at least 45 mgs of pectin, more preferably at least 50 mgs of pectin and most preferably at least 55mgs of pectin per lozenge provide the desired performance.

There is clearly a relationship between the concentration of pectin in the mixture and the amount of pectin in each final lozenge. Given the inherent difficulties in increasing the concentration of pectin in the batches, any increase in pectin required per lozenge would require an increase in lozenge size to achieve.

The sweetener in the lozenges of the present invention comprises between 78 -88% by weight of the lozenge, more preferably between 80 and 86 weight % and most preferably between 81 and 84% by weight of the lozenge.

The sweetener may comprise any sweetening agents known in the art. Sweeteners help to encourage the consumption of the lozenges by making them pleasant to taste.

The sweeteners of the present invention may be natural or artificial "sugars". Artificial sugars can be beneficial to those seeking relief from a sore throat while maintaining control over their calorie intake.

Preferably the sweetener comprises one or more sugars.

The sugars may be chosen from a list comprising fructose, mannose, lactose, galactose, sucrose, glucose, dextrose and mixtures thereof.

The sugars may be in their natural or unnatural forms, or mixtures thereof.

It is particularly preferred that the sweetener of the present invention comprises a mixture of glucose and sucrose.

The glucose and sucrose may be present in any weight ratio. Preferably the two sugars may be used in a weight ratio of 80:20 to 20:80, more preferably a ratio of 60:40 to 40:60 and most preferably a ratio of 55:45 to 45:55.

In a preferred lozenge composition, sucrose and glucose are used in an approximately 1:1 weight ratio.

The sugars may be used in any form. From granules of crystallised sugar, aqueous solutions, to natural syrups.

A particularly preferred source of glucose for the present invention is corn syrup. A particularly preferred source of sucrose is granulated sucrose and water solutions of sucrose.

Acid is used in the formulation to adjust the pH to aid the solidification process. Any suitable acid could be used for this purpose.

A particularly preferred acid source is citric acid or citric acid salts or mixtures thereof.

The acid is utilised from 0.1 to 1.5 % by weight of the lozenge, more preferably between 0.05 % and 1.0 % and most preferably around 1 % by weight.

The amount of water present in the lozenge can range from 10 to 19 % by weight. More preferably the water may be present between 11 and 16 % by weight and most preferably between 14 and 16 % by weight.

A more preferred lozenge of the present invention comprises or consists essentially of:

**Table 2**

| **Ingredient** | **% Weight** |
|---|---|
| Pectin | 1.5-1.7 % |
| Sweetener | 81-84 % |
| Acid | 0.5-1.0 % |
| Water | 11-16 % |

Further optional minor ingredients include flavourings and if necessary, colourings. Optional minor ingredients will make up less than 1% by weight of the lozenge. More preferably the minor ingredients will make up less than 0.75% by weight and most preferably less than 0.5% by weight.

The flavorings used may be natural or artificial or a mixture of the two. There are no limitations on the choice of flavorings for the present invention.

It is preferred to use natural flavors. Particularly preferred flavours are natural products such as honey or mint extracts.

The lozenges of the present invention are formulated to provide relief to sore throats as described using pectin as a sole active. However, it may be desirable to increase performance of the lozenges through the addition of at least one further active ingredient.

Any further active ingredient may be used. It is particularly preferred that additional actives may comprise anaesthetics, antitussives and decongestants and mixtures thereof as required.

Specific APIs that are of specific relevance to the present invention include menthol, benzocaine, guaifenesin, acetaminophen, phenylephrine HCl, Dextromethorphan HBr and mixtures of these.

The invention is not limited to these actives and the skilled person can consider other examples.

A particularly preferred lozenge of the present invention comprises or consists essentially of:

**Table 3**

| **Ingredient** | **% Weight** | **Mass** |
|---|---|---|
| Pectin | 1.60 % | 57 mg |
| Sweetener (Sucrose/Corn Syrup 1:1) | 81.9 % | 2949 mg |
| Citric Acid/salts | 0.9 % | 32 mg |
| Water | 15.2 % | 548 mg |
| Flavor | 0.4 % | 14 mg |
| **Total:** | | 3.6 grams |

### Method of manufacture

The method of manufacture is not critical to the present invention. The skilled artisan will be familiar with the general process of preparing lozenges.

US 5,932,273 and US 2010/0226904 A1 disclose example methods of manufacture for the making of lozenges.

The essentials of the process require getting a mixture of all the ingredients in solution (water) at an elevated temperature and then driving off the excess water until the formula reaches the desired levels of moisture. Finally, the formulation is allowed to cool in moulds until it sets.

The precise order of addition of the ingredients to the initial solution is not critical to the present invention. Although it is typical to add the acid component at the end as this drives the gelation of the pectin. The precise batch temperatures will vary by manufacturing equipment used.

Applicants have found that temperatures of over 110°C for the mixing and moisture optimisation process, 90°C for the transfer to moulds, and room temperatures for drying are optimal.

### Hardness Test:

The lozenges of the present invention are intermediate between the hardness of a classic hard-boiled sugar lozenge and a gummy. This particular hardness range has been found by Applicant to optimise consumption time in the mouth by discouraging both chewing and crunching of the lozenges.

The following texture profile analysis (TPA) hardness test was used to test the lozenges and to attain the correct level of hardness.

The TPA method is a very commonly used method for characterizing food properties. See https://texturetechnologies.com/resources/texture-profile-analysis#settings-and-standards for more information).

The method double compresses the sample and measure properties such as hardness, stickiness, resilience etc. For the purposes of the present invention the test was used to test for hardness.

The test has several defined steps. The key set points for the hardness test used herein are:
**Probe:** A 40mm flat probe as it was much larger than the sample area being tested. This allows for a lot of room for human error in sample positioning (i.e., As long as the sample is entirely underneath the probe it will test repeatably.)

**Compression and withdrawal speed:** 2mm/s was used as an appropriate testing speed. A wide range of speeds will work. However, too fast and the resolution of the peak may not be clear due to sampling rate. Sampling slower is possible but these tests are usually conducted on a large number of samples so overall testing time increases. 2mm/s provides a balance between resolution and testing time.

**Compression distance:** compression distance is set to a strain%. A height calibration is required for strain measures. The probe travels downward at 3mm/s "looking" for the sample. Once the load cell receives 10g of force the test is initiated.

The probe calculates sample height from the height calibration and proceeds to compress the sample to 50% of its total height. Higher strain % are used to simulate sample chewing. A strain of 50% was chosen as it provided accurate and repeatable hardness readings without exceeding the load cell capacity.

Repeatability - All results shown are an average of at least 10 tests.

### Results of hardness testing

Lozenges of the present invention, (table 3 formulation) were tested against 3 commercial pectin (low level) containing lozenges that were available for sale for comparison.

The results on table 4 clearly show that the lozenges of the invention are intermediate in hardness between a soft gummy and a hard candy. (The Ludens sample was too hard to register a score in this test, the actual hardness value was off the scale.)

**Table 4**

| **Pectin product tested** | **Hardness value (g)** | **Standard deviation** |
|---|---|---|
| Lozenge of the invention (Table 3) | 11,873 (g) | (+/-) 1166 |
| Welchs (soft gummy) | 2,432 (g) | (+/-) 346 |
| Commercial sample | | |
| Zzz Quill (soft gummy) | 6,125 (g) | (+/-) 696 |
| Commercial sample | | |
| Ludens (hard candy) | >50,000 (g) | N/A |
| Commercial sample | | |

It is preferred that the lozenges of the present invention have a hardness value (according the TPA test outlined above with a 50% strain) of between 8,000 and 20,000 g, more preferably between 9,000 and 15,000 g and most preferably between 10,000 and 13,000 g.

A particularly preferred hardness for lozenges of the present invention is around 12,000 g.

This balance allows for high pectin content and encourages the correct consumer behaviour to optimise the soothing benefit.

For optimum performance the lozenges of the present invention may slowly dissolve in the mouth and release pectin in the mouth over a period of a minimum of 5 minutes. Preferably the lozenges should dissolve over a period of a minimum of 10 minutes and most preferably for a minimum of 12 minutes.

## Claims

1. A lozenge comprising:
1.4-1.8% by weight pectin;
78-88% by weight sweetener;
0.1-1.5% of an organic acid; and
10-19% by weight of water.

2. The lozenge of claim 1, wherein (i) the lozenge is free of gelling agents and rheology modifying agents; or (ii) the lozenge is free of gelatin and starch.

3. The lozenge of claim 1, wherein the lozenge has a total weight of between 2 and 6 grams.

4. The lozenge of claim 1, wherein the lozenge comprises at least 40 mg of pectin.

5. The lozenge of claim 1, wherein (i) the sweetener comprises between 82-86% by weight of the lozenge; or (ii) the sweetener is an artificial sweetener; or (iii) the sweetener comprises one or more of fructose, sucrose, lactose, glucose and mixtures thereof.

6. The lozenge of claim 1, wherein the sweetener is a combination of sucrose and corn syrup, particularly wherein the sucrose and corn syrup are present in a weight ratio of 20:80 to 80:20.

7. The lozenge of claim 1 wherein the organic acid comprises between 0.5-1% by weight of the lozenge.

8. The lozenge of claim 1, wherein the organic acid comprises citric acid, salts of citric acid and mixtures thereof.

9. The lozenge of claim 1, wherein the water comprises between 12-17% by weight.

10. The lozenge of claim 1, further comprising a flavoring, a dye, a preservative, or mixtures thereof.

11. The lozenge of claim 1, wherein the lozenge has a hardness of between 8000 and 20,000 grams as measured by a TPA test.

12. The lozenge of claim 1 further comprising an additional active ingredient selected from a group consisting of menthol, guaifenesin, acetaminophen, benzocaine, phenylephrine HCl, dextromethorphan HBr, and mixtures thereof.

13. A lozenge according to claim 1 consisting essentially of:
1.4-1.8% by weight pectin;
81-83% by weight sucrose and glucose;
14-17% by weight water;
0.9-1.10% by weight citric acid and/or citric acid salts and/or mixtures thereof; and
0.1 -0.5% by weight flavorings.

14. The lozenge of claim 13, wherein a weight ratio of sucrose to glucose is between 60:40 and 40:60.

15. The lozenge of claim 13, wherein the lozenge has a total weight of between 3 and 4 grams.

16. The lozenge of Claim 1 for use in a method of treating sore throats comprising: applying the lozenge to an oral cavity of a patient in need; and dissolving the lozenge over a period of at least 5 minutes.

17. The lozenge of claim 16 for use in a method of treating sore throats, wherein the lozenge dissolves over a period of at least 10 minutes.

## Patentansprüche

1. Lutschtablette, umfassend:
1,4 bis 1,8 Gew.-% Pektin;
78-88 Gew.-% Süßstoff;
0,1-1,5 % einer organischen Säure; und
10-19 Gew.-% Wasser.

2. Lutschtablette gemäß Anspruch 1, worin (i) die Lutschtablette frei von Geliermitteln und Rheologiemodifizierenden Mittel ist; oder (ii) die Lutschtablette frei von Gelatine und Stärke ist.

3. Lutschtablette gemäß Anspruch 1, worin die Lutschtablette ein Gesamtgewicht zwischen 2 und 6 Gramm aufweist.

4. Lutschtablette gemäß Anspruch 1, worin die Lutschtablette mindestens 40 mg Pektin umfasst.

5. Lutschtablette gemäß Anspruch 1, worin (i) der Süßstoff zwischen 82-86 Gew.-% der Lutschtablette ausmacht; oder (ii) der Süßstoff ein künstlicher Süßstoff ist; oder (iii) der Süßstoff eines oder mehrere von Fructose, Saccharose, Lactose, Glucose und Mischungen hiervon umfasst.

6. Lutschtablette gemäß Anspruch 1, worin der Süßstoff eine Kombination von Saccharose und Maissirup ist, insbesondere worin die Saccharose und der Maissirup in einem Gewichtsverhältnis von 20:80 bis 80:20 vorliegen.

7. Lutschtablette gemäß Anspruch 1, worin die organische Säure zwischen 0,5-1 Gew.-% der Lutschtablette ausmacht.

8. Lutschtablette gemäß Anspruch 1, worin die organische Säure Zitronensäure, Salze von Zitronensäure und Mischungen hiervon umfasst.

9. Lutschtablette gemäß Anspruch 1, worin das Wasser 12-17 Gew.-% ausmacht.

10. Lutschtablette gemäß Anspruch 1, ferner umfassend einen Geschmacksstoff, einen Farbstoff, einen Konservierungsstoff oder Mischungen hiervon.

11. Lutschtablette gemäß Anspruch 1, worin die Lutschtablette eine Härte zwischen 8.000 und 20.000 Gramm aufweist, gemessen mit einem TPA-Test.

12. Lutschtablette gemäß Anspruch 1, ferner umfassend einen zusätzlichen Wirkstoff, ausgewählt aus der Gruppe bestehend aus Menthol, Guaifenisin, Paracetamol, Benzocain, Phenylephrin HCl, Dextromethorphan HBr und Mischungen hiervon.

13. Lutschtablette Gemäß Anspruch 1, im Wesentlichen bestehend aus:
1,4 bis 1,8 Gew.-% Pektin;
81-83 Gew.-% Saccharose und Glucose;
14-17 Gew.-% Wasser;
0,9-1,10 Gew.-% Zitronensäure und/oder
Zitronensäuresalze und/oder Mischungen hiervon; und
0,1-0,5 Gew.-% Geschmacksstoffe.

14. Lutschtablette gemäß Anspruch 13, worin das Gewichtsverhältnis von Saccharose und Glucose zwischen 60:40 und 40:60 liegt.

15. Lutschtablette gemäß Anspruch 13, worin die Lutschtablette ein Gesamtgewicht zwischen 3 und 4 Gramm aufweist.

16. Lutschtablette gemäß Anspruch 1 zur Verwendung in einem Verfahren zur Behandlung von Halsschmerzen, umfassend:
Einbringen der Lutschtablette in die Mundhöhle eines Patienten, der dessen bedarf; und Auflösen der Lutschtablette über eine Zeitspanne von mindestens 5 Minuten.

17. Lutschtablette gemäß Anspruch 16 zur Verwendung in einem Verfahren zur Behandlung von Halsschmerzen, worin die Lutschtablette sich über eine Zeitspanne von mindestens 10 Minuten löst.

## Revendications

1. Pastille comprenant:
1,4 - 1,8 % en poids de pectine;
78 - 88 % en poids d'édulcorant;
0,1 - 1,5 % d'un acide organique; et
10 - 19 % en poids d'eau.

2. Pastille selon la revendication 1, où (i) la pastille est exempte d'agents gélifiants et d'agents modifiant la rhéologie; ou (ii) la pastille est exempte de gélatine et d'amidon.

3. Pastille selon la revendication 1, où la pastille a un poids total entre 2 et 6 grammes.

4. Pastille selon la revendication 1, où la pastille comprend au moins 40 mg de pectine.

5. Pastille selon la revendication 1, où (i) l'édulcorant représente entre 82 et 86 % en poids de la pastille; ou (ii) l'édulcorant est un édulcorant artificiel; ou (iii) l'édulcorant comprend un ou plusieurs parmi le fructose, le saccharose, le lactose, le glucose et des mélanges de ceux-ci.

6. Pastille selon la revendication 1, où l'édulcorant est une combinaison de saccharose et de sirop de maïs, en particulier où le saccharose et le sirop de maïs sont présents dans un rapport pondéral de 20:80 à 80:20.

7. Pastille selon la revendication 1, où l'acide organique représente entre 0,5 et 1 % en poids de la pastille.

8. Pastille selon la revendication 1, où l'acide organique comprend de l'acide citrique, des sels d'acide citrique et des mélanges de ceux-ci.

9. Pastille selon la revendication 1, où l'eau représente entre 12 et 17 % en poids.

10. Pastille selon la revendication 1, comprenant en outre un arôme, un colorant, un conservateur ou des mélanges de ceux-ci.

11. Pastille selon la revendication 1, où la pastille a une dureté entre 8 000 et 20 000 grammes telle que mesurée par un test TPA.

12. Pastille selon la revendication 1 comprenant en outre un ingrédient actif supplémentaire choisi dans un groupe consistant en le menthol, la guaifénésine, l'acétaminophène, la benzocaïne, la phényléphrine HCl, le dextrométhorphane HBr et des mélanges de ceux-ci.

13. Pastille selon la revendication 1 consistant essentiellement en:
1,4 - 1,8 % en poids de pectine;
81 - 83 % en poids de saccharose et de glucose;
14 - 17 % en poids d'eau;
0,9 - 1,10 % en poids d'acide citrique et/ou de sels d'acide citrique et/ou des mélanges de ceux-ci; et
0,1- 0,5 % en poids d'arômes.

14. Pastille selon la revendication 13, où un rapport pondéral du saccharose au glucose est entre 60:40 et 40:60.

15. Pastille selon la revendication 13, où la pastille a un poids total entre 3 et 4 grammes.

16. Pastille selon la revendication 1 destinée à être utilisée dans un procédé de traitement des maux de gorge comprenant: l'application de la pastille à une cavité buccale d'un patient qui en a besoin; et la dissolution de la pastille sur une période d'au moins 5 minutes.

17. Pastille selon la revendication 16 destinée à être utilisée dans un procédé de traitement des maux de gorge, où la pastille se dissout sur une période d'au moins 10 minutes.
